(19) Europäisches Patentamt
European Patent Office
Office européen des brevets

(11) Veröffentlichungsnummer: **0 028 723**
**B1**

# (12) EUROPÄISCHE PATENTSCHRIFT

(45) Veröffentlichungstag der Patentschrift: **26.05.82**

(51) Int. Cl.³: **C 07 C 109/087**

(21) Anmeldenummer: **80106285.2**

(22) Anmeldetag: **16.10.80**

## (54) Verfahren zur Herstellung von N,N'-Diformylhydrazin.

(30) Priorität: **26.10.79 DE 2943264**

(43) Veröffentlichungstag der Anmeldung: **20.05.81 Patentblatt 81/20**

(45) Bekanntmachung des Hinweises auf die Patenterteilung: **26.05.82 Patentblatt 82/21**

(84) Benannte Vertragsstaaten: **AT BE CH DE FR GB IT LI NL**

(56) Entgegenhaltungen:

**JOURNAL OF THE AMERICAN CHEMICAL SOCIETY Band 77, 1955 Columbus C. AINSWORTH et al. "Isomeric and Nuclear-substituted B-Aminoethyl-1,2,4-triazoles" Seiten 621 bis 624**

**"Soviet Inventions Illustrated" Week B 26, 8. August 1979 Section B 05 C 03**

**Chemical Abstracts Band 53, Nr. 12, 25. Juni 1959 Columbus, Ohio, USA R.F.W. RÄTZ et al. "Products from reaction of hydrazine and thinoöxamic acid and their conversion into heterocyclic compounds" Spalten 11398h-i und 11399a-b**

(73) Patentinhaber: **HOECHST AKTIENGESELLSCHAFT, Postfach 80 03 20, D-6230 Frankfurt/Main 80 (DE)**

(72) Erfinder: **Knorr, Harald, Dr., Hans-Fischer-Strasse 6, D-8906 Gersthofen (DE)**

EP 0 028 723 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von N,N'-Diformylhydrazin

N,N'-Diformylhydrazin der Formel

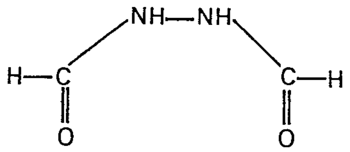

ist ein Zwischenprodukt bei der Herstellung von 1,2,4-Triazol, welches seinerseits ein wichtiges Ausgangsmaterial für herbizide und fungizide Produkte darstellt.

N,N'-Diformylhydrazin wird im allgemeinen durch Umsetzen von Hydrazinhydrat mit Formamid bei erhöhter Temperatur erhalten [C. Ainsworth und R.G. Jones, J. Amer. Chem. Soc. 77, 621-4 (1955)]. Nach einer Reaktionszeit von etwa zwei Stunden fällt ein Reaktionsgemisch an, welches zu etwa 80% aus dem gewünschten Produkt besteht. Um dieses zu gewinnen, ist es erforderlich, Alkohol zuzusetzen, wobei Nebenprodukte, wie z.B. N-Aminotriazol, in Lösung gehen. Als besonderer Nachteil dieses Verfahrens muss diese Reinigungsstufe mit dem zwingenden Zusatz eines Lösungsmittels angesehen werden, welches ja vor allem beim Arbeiten in technischen Mengen wieder regeneriert werden muss. Darüber hinaus sind Nebenprodukte in einer Grössenordnung von 20% - wobei deren Menge bei Vergrösserung der Ansätze zunimmt -, welche nicht mehr verwertbar sind und somit verloren gehen, bei einem technischen Verfahren untragbar.

Es bestand daher die Aufgabe, eine verbesserte Methode zur Synthese des N,N'-Diformylhydrazins zu finden, um eine Übertragung in den technischen Masstab zu gewährleisten. Eine möglichst vollständige Umsetzung zu erzielen, wäre zudem eine wichtige Voraussetzung für eine folgende Weiterverarbeitung zum Triazol und würde kostenspielige Reinigungen und Trennungen von Stoffgemischen vermeiden.

Es wurde gefunden, dass die Herstellung von N,N'-Diformylhydrazin in sehr hohen Ausbeuten möglich ist, wenn man dafür sorgt, dass der bei der Umsetzung von Hydrazinhydrat mit Formamid freiwerdende Ammoniak schnell aus dem System entfernt wird.

Die Erfindung betrifft somit ein verbessertes Verfahren zur Herstellung von N,N'-Diformylhydrazin der Formel

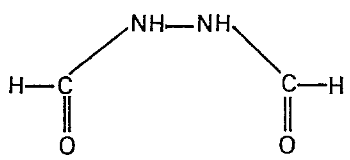

durch Umsetzen von Formamid mit Hydrazinhydrat, welches dadurch gekennzeichnet ist, dass man zunächst Formamid und Hydrazinhydrat im Molverhältnis von 2:1,2 bis 2:0,6 bei einer Temperatur von 5 bis 25°C mischt und diese Mischung bei der gleichen Temperatur unter vermindertem Druck so lange sich selbst überlässt, bis die Ammoniakabspaltung beendet zu sein scheint, sodann die Temperatur auf 80 bis 120°C erhöht und ebenfalls unter vermindertem Druck 1 bis 3 Stunden nachreagieren lässt und schliesslich unter weiterer Druckreduzierung Wasser sowie nicht umgesetztes Ausgangsmaterial abdestilliert.

Dass eine Herstellungmethode unter Anlegen von Vakuum von Vorteil sein könnte, war nicht zu erwarten, da vielmehr befürchtet werden musste, dass unter diesen Reaktionsbedingungen das Hydrazinhydrat als leicht flüssiger Bestandteil abdestillieren würde, bevor es mit dem Formamid reagieren kann.

Formamid und Hydrazinhydrat werden im Molverhältnis von 2:1,2 bis 2:0,6, vorzugsweise 2:0,8 bis 2:1,0 eingesetzt. Das Hydrazinhydrat kann auch in Form von wässrigen, im allgemeinen ca. 80%igen Lösungen Verwendung finden.

Die Umsetzung wird in zwei Stufen durchgeführt. In der ersten Stufe lässt man Formamid und Hydrazinhydrat bei Temperaturen von 5 bis 25°C miteinander reagieren, wobei man Vakuum anlegt. Wenn die Ammoniakabspaltung beendet zu sein scheint, was im allgemeinen nach 1 bis 2 Stunden der Fall ist, wird unter Beibehaltung des Vakuums auf 80 bis 120°C, vorzugsweise auf ca. 100°C aufgeheizt und bei dieser Temperatur in einer zweiten Reaktionsstufe die Umsetzung vervollständigt. Hierfür werden im allgemeinen nochmals 1 bis 3, vorzugsweise 1 bis 2 Stunden benötigt. Eine bevorzugte Arbeitsweise besteht darin, in der ersten Reaktionsstufe bei 70 bis 600 mbar und in der zweiten Reaktionsstufe bei 5 bis 70 mbar umzusetzen. Nach Beendigung der Reaktion wird schliesslich unter weiterer Reduzierung des Druckes Wasser und nicht umgesetztes Ausgangsmaterial abdestilliert. Zurück bleibt praktisch reines N,N'-Diformylhydrazin in Ausbeuten von über 95%, bezogen auf Hydrazinhydrat.

Die nachfolgenden Beispiele dienen der weiteren Erläuterung des Verfahrens.

Beispiel 1

10 Mol Formamid (450 g) und 250 ml Hydrazinhydrat (ca. 80%ig in Wasser) werden bei Raumtemperatur zusammengegeben. Unter gutem Rühren wird sofort Vakuum angelegt, welches sich auf ca. 150 mbar einstellt. Man belässt eine Stunde bei Raumtemperatur, wobei sich der Druck auf 70 mbar reduziert, erwärmt dann auf 100°C und hält noch 1,5 Stunden im Vakuum. Nach dieser Zeit hat sich ein Druck von 13 mbar eingestellt. Bei diesem wird schliesslich Wasser und nicht umgesetztes Ausgangsmaterial abdestilliert. Nach dem Trocknen des verbliebenen Rückstandes im Vakuum bei 115°C erhält man 351 g N,N'-Diformylhydrazin (97,3%, bezogen auf 100%iges Hydrazinhydrat) vom Schmelzpunkt 159°C (Lit.: 159 bis 160°C oder 160°C).

Vergleichsbeispiel

Die Komponenten werden wie in Beispiel 1 zusammengegeben und dann 2 Stunden bei 100°C gehalten, ohne dass Vakuum angelegt wird. Man destilliert dann die flüchtigen Bestandteile ab und fügt 1000 ml Ethanol zu. Beim Abkühlen kristallisieren 273 g N,N'-Diformylhydrazin vom Schmp. 158°C aus. Im Filtrat weist man u.a. 7,7% N,-Aminotriazol und 12,3% N,N'-Diformylhydrazin sowie 36,1% Formamid nach. Die Gesamtmenge N,N'-Diformylhydrazin beträgt demnach 294 g (81,5%, bezogen auf Hydrazin).

Beispiel 2

In einem labormischer von 5 l Inhalt (Loedige-Mischer) werden 1375 ml ca. 80%iges Hydrazinhydrat und 2250 g Formamid bei 20°C zusammengegeben. Sodann wird Vakuum angelegt, wobei sich zunächst ein Druck von etwa 150 mbar einstellt. Das sich entwickelnde Ammoniakgas wird abgezogen und kondensiert. Man lässt 1 Stunde weiterreagieren, in deren Verlauf sich der Druck auf etwa 100 mbar erniedrigt und heizt sodann im Ölbad auf 100°C auf. Hierbei stellt sich zunächst ein Druck von ca. 70 mbar ein, der schliesslich bis auf 20 mbar abfällt. Man lässt 2 Stunden bei dieser Temperatur, wobei Wasser abdestilliert. Dann steigert man die Ölbadtemperatur auf 140°C und destilliert weiteres Wasser und überschüssiges Formamid innerhalb von 5 Stunden bis zu einem Endvakuum von 4 mbar ab. Die Gesamtmenge des Destillates beträgt rund 287 g.

Es werden 1964 g N,N'-Diformylhydrazin vom Schmp. 157°C in Form einer farblosen, pulverigen Masse erhalten.

## Patentansprüche

1. Verfahren zur Herstellung von N,N'-Diformylhydrazin der Formel

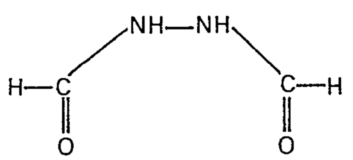

durch Umsetzen von Formamid mit Hydrazinhydrat, dadurch gekennzeichnet, dass man zunächst Formamid und Hydrazinhydrat im Molverhältnis 2:1,2 bis 2:0,6 bei einer Temperatur von 5 bis 25°C mischt und diese Mischung bei der gleichen Temperatur unter vermindertem Druck so lange sich selbst überlässt, bis die Ammoniakabspaltung beendet zu sein scheint, sodann die Temperatur auf 80 bis 120 öht und ebenfalls unter vermindertem Druck 1 bis 3 Stunden nachreagieren lässt und schliesslich unter weiterer Druckreduzierung Wasser sowie nicht umgesetztes Ausgangsmaterial abdestilliert.

2. Verfahren nach Anspruch 1, dadurch gekennzeichnet, dass in der ersten Reaktionsstufe bei 70 bis 600 mbar und in der zweiten Reaktionsstufe bei 5 bis 70 mbar gearbeitet wird.

## Claims

1. A process for the preparation of N,N'-diformylhydrazine of the formula

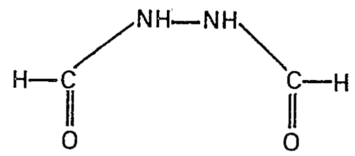

by reacting formamide and hydrazine hydrate, which comprises mixing first formamide and hydrazine hydrate in a molar ratio of from 2:1 to 2:0.6 at a temperature of from 5 to 25°C, and abandoning this mixture at the same temperature under reduced pressure until splitting-off of ammonia seems to be terminated, raising then the temperature to 80–120°C and allowing the reaction to continue for 1 to 3 hours also under reduced pressure, and finally distilling off water and unreacted starting material under a pressure reduced still further.

2. The process as claimed in claim 1, which comprises operating in the first step at 70 to 600 mbar, and at 5 to 70 mbar in the second reaction step.

## Revendications

1. Procédé de préparation de la N,N'-diformylhydrazine, corps qui répond à la formule suivante:

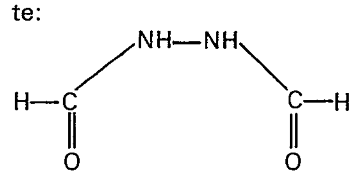

par réaction du formamide avec l'hydrate d'hydrazine, procédé caractérisé en ce qu'on mélange d'abord le formamide et l'hydrate d'hydrazine dans un rapport molaire de 2:1,2 à 2:0,6, à une température de 5 à 25°C, on laisse reposer ce mélange à la même température, sous pression réduite, jusqu'à ce que le dégagement d'ammoniac semble terminé, puis on élève la température à 80–120°C, on laisse réagir également sous pression réduite pendant 1 à 3 heures et enfin, tout en continuant à abaisser la pression, on chasse par distillation l'eau ainsi que la matière de départ qui n'a pas réagi.

2. Procédé selon la revendication 1, caractérisé en ce qu'on opère sous une pression de 70 à 600 mbar au cours de la première étape réactionnelle, et de 5 à 70 mbar au cours de la seconde étape réactionnelle.